# EUROPEAN PATENT APPLICATION

(11) **EP 4 716 226 A1**
(43) Date of publication of application: **25.03.2026**
(21) Application number: 25203228.9
(22) Date of filing: 19.09.2025
(51) Int. Cl.: H04N 23/51, A61F 9/08, G03B 17/04, H04N 23/56, H04N 23/57, H04N 23/69

(54) **WIFI COUPLED LOW VISION CAMERA**

(30) Priority: 24.09.2024 US 202463698408 P; 19.05.2025 US 202519212335
(71) Applicant: Eschenbach Optik GmbH, 90429 Nürnberg (DE)
(72) Inventor: Bollmann, Andreas, 90429 Nürnberg (DE); Apel, Nicky, 91207 Lauf an der Pegnitz (DE); Alles, Mark, 90602 Pyrbaum (DE)
(74) Representative: Rau, Schneck & Hübner Patentanwälte Rechtsanwälte PartGmbB

(57) **Abstract**

A low vision aid system including a camera module to be used with a mobile device, including a macro camera configured to focus on near-field objects that is integrated within the low vision aid camera module and an illumination source structured to illuminate a field of view associated with the macro camera, a battery power supply and a wireless communication module. The wireless communication module establishes an isolated Wi-Fi connection to the mobile device. The isolated Wi-Fi connection prevents the mobile device from communicating over other Wi-Fi networks while the isolated Wi-Fi connection is maintained. The wireless communication module receives, via the isolated Wi-Fi connection, instructions from the mobile device, that are associated with operation of the macro camera, and sends via the isolated Wi-Fi connection, a video bitstream to the mobile device based on the instructions.

## Description

### CLAIM TO PRIORITY

This application claims the benefit of United States Provisional Application 63/698,408, filed September 24, 2024, entitled "Wi-fi Coupled Low Vision Camera", as well as the benefit of United States Utility Application 19/212,335, filed May 9, 2025, entitled "Wi-fi Coupled Low Vision Camera", which are hereby fully incorporated herein by reference.

### TECHNICAL FIELD

The present disclosure relates generally to a low vision aid camera module for a smartphone or other mobile electronic device. More particularly, embodiments facilitate control of digital imaging capabilities of the low vision aid camera module via an isolated Wi-Fi connection.

### BACKGROUND

Mobile devices have become ubiquitous tools for communication, information access, and daily tasks. However, for individuals with low vision, using these devices can present significant challenges. While smartphones and tablets offer a range of accessibility features, such as screen magnifiers, voiceover functionality, and high-contrast modes, these software-oriented tools often fall short in addressing the specific needs of users with low vision. This is particularly evident in the context of written or printed materials in varied lighting conditions.

One shortcoming of traditional mobile accessibility options is an inability to adequately enlarge printed materials and objects in the user's surroundings. While some third-party applications attempt to bridge the gap by offering enhanced magnification or contrast options, these solutions are often limited by the hardware capabilities of the device. For instance, the quality of zoomed images can be hindered by the camera's resolution, and the app-based solutions might suffer from lag or poor usability due to processing or networking limitations. Additionally, these features can be cumbersome to use, requiring multiple gestures or settings adjustments that can be frustrating for users with limited vision.

Recognizing the limitations of mobile devices, many individuals with low vision turn to independent low vision aids. These devices, such as electronic magnifiers and wearable vision aids, are specifically designed to enhance visual input for the user. However, independent low vision aids face their own set of challenges. Independent low vision aids are often bulky, making the devices less portable and convenient than the slim, multifunctional design of mobile devices, such as a smartphone. Independent low vision aids also tend to be expensive, limiting accessibility for many users. Additionally, because these devices operate separately from smartphones, users are required to carry multiple devices, manage different battery life cycles, and switch between tools depending on the task at hand. This lack of integration can be cumbersome, particularly for users who are already dealing with the challenges of low vision.

Moreover, the user experience of independent low vision aids often adds complexity. Low vision aids often have steep learning curves to operate effectively, with multiple settings and modes that can be overwhelming for users. The need to regularly update software or perform maintenance on the device can further complicate usability.

Therefore, there is a need for systems and methods that integrate with mobile devices to supplement low-vision capabilities.

### SUMMARY

Embodiments described or otherwise contemplated herein substantially meet the aforementioned needs of the industry. Embodiments described herein include systems and methods for supplementing the near field imaging capabilities of a mobile device over an isolated Wi-Fi connection.

In an example embodiment, a low vision camera module for use with a mobile device includes a macro camera, a light source, a battery power supply, and a wireless communication module. The macro camera is integrated within the low vision aid camera module and configured to focus on near-field objects. The light source is positioned and structured to illuminate a field of view associated with the macro camera. The wireless communication module is configured to: establish an isolated Wi-Fi connection to the mobile device, preventing the mobile device from communicating over other Wi-Fi networks while the isolated Wi-Fi connection is maintained. The wireless communication module can, via the isolated Wi-Fi connection, receive instructions associated with operation of the macro camera, and send a video bitstream to the mobile device based on the instructions.

According to an example embodiment of the invention, an app on the associated smartphone initializes an exclusive encrypted Wi-Fi connection between the smart phone and the smart phone camera module. The app uses, for example, an API with iOS to ask the operating system to connect to the stored Wi-Fi exclusively associated with the camera module. At the same time the app shuts off connection with any other Wi-Fi networks at least for the duration of the connection to the exclusive Wi-Fi network. Accordingly, all communication over the Wi-Fi between the smart phone or digital device and the camera module is private so long as the password is not compromised.

Using a wireless connection between the smart phone and the camera module allows independence from geometric dependencies. This allows the use of the camera module associated camera which is optimized for reading and the user group of low-vision and/or elderly patients. The use of the independent camera associated with the camera module enables delivery of a video stream with consistent characteristics independent of the phone's or digital device's built-in camera. This avoids a problem of having to deal with the different specifications of different cameras in different phone models. According to some example embodiments, a wired connection or connections such as a USB or lightning cable may be utilized to couple the smartphone or handheld device to the camera module for example to facilitate large amounts of data transfer between the devices.

According to an example embodiment of the invention, incorporated image signal processing in the camera module can create, for example, a contrast-enhanced two-color image which is especially useful in an electronic reading aid for certain low-vision patients. Further the illumination system built into the camera module combination provides optimized additional illumination to support the illumination needs of the camera incorporated into the camera module. In general, this illumination may be more than that required for the use of a standard smart device mobile camera.

According to example embodiments of the invention, the camera objective incorporated into the camera module is centrally located in the relative to the screen display of the associated smart phone or digital device. Central location of the camera objective facilitates intuitive alignment by the user between the target, the camera objective and the viewer's eye. The center of the device display tends to be along the optical axis of the viewer's eye. Accordingly, the viewer's eye, the device display and the camera objective are oriented substantially on a single optical axis.

According to example embodiments of the invention, the camera has a short minimum object distance (MOD) or working distance to facilitate placement of the device close to the text or other object to be viewed. For example, the MOD may be in a range of one half to six inches or 1.25 to 10 centimeters.

Further, according to example embodiments of the invention, the camera has a large field of view at the above-mentioned minimum object distance. In addition, the optics of the camera are optimized to have a large depth of focus thereby delivering sharp images over a range of object distances from the lens. According to a further example embodiment, the image processing or postprocessing is optimized for this optical geometric scenario thus facilitating the special needs of low-vision users.

According to example embodiments of the invention certain features and functions are built into the camera and features of the camera module. These functions include but are not limited to the capability to manipulate light in conjunction with readability improvements, contrast manipulation, color manipulation to assist low-vision patients and users. In addition, other image manipulations may be applied and are included in the camera module rather than in an app on the phone. For example, the image processing manipulations may include multiple false color modes that facilitate optimal contrast between written material and its background for various impaired vision related conditions. According to an example embodiment, up to fourteen false color modes may be provided. These features are built into the camera module. Features are done by the camera module such as exposure management, color correction, reducing image noise, sharpening and so on. In the case of the example embodiments of the invention, full access is provided to the cameras ISP and therefore all parameters can be optimized for a low-vision situation. This largely eliminates the problem of needing to access the image processing features of an internal phone feature.

The integrated camera module camera includes automatic gain control. The camera may also take advantage of a TellMe function which utilizes the smart phones vision framework for optical character recognition. The integrated camera processes the video data, command sent from the smart phone via the app and Wi-Fi credentials as it serves as the Wi-Fi access point. The app on the smart phone processes the same data as receives and displays the video stream, creates and sends commands from user inputs and instructs the phone to connect to the Wi-Fi of the camera exclusively.

According to an example embodiment of the invention the camera in the camera module compresses the video stream using H. 264 before sending the video stream via Wi-Fi to the following. According to example embodiments of the invention the whole process of creating a video steam stream from raw sensor data can be accomplished by video data manipulation within the camera module.

According to example embodiments, the camera module can be attached to a smart phone or other digital device at least 2 ways. According to one example embodiment a custom fit integrated case that holds both a smart phone and the camera module together can be utilized. According to another example embodiment a magnetic universal stand allows almost any smart phone or digital device to be magnetically attached to the camera model for use as an integrated unit.

An app that can be installed on the phone facilitates communication between the phone or other digital device with the camera module and turns the phone and camera module combination into a full featured video magnifier. According to an example embodiment this provides a full HD image through the display on the phone or visual device with magnification ranging from 3.3 times to 15 times as well as access to features available in a typical dedicated video magnifier that are helpful to those with vision loss.

According to another example embodiment LED illumination is incorporated into the camera model and can be adjusted or turned off facilitating a glare free image. In addition, there are for example 14 color mode options from which to choose.

According to another example embodiments there is also an adjustable reading line or blinds, a read aloud function and a customizable menu all of which contribute to a more comfortable and effective viewing experience for those with a vision impairment. According to an example embodiment the camera module also includes a foldout stand which holds the camera module and smart phone or digital device in a comfortable ergonomic angle so that it can be used for extended reading tasks. In addition, the camera located under the center of the smart phone or other handheld digital device automatically swivels into a reading position when the stand is folded out to mitigate on screen distortion and orientation issues. For example, this swiveling feature mitigates keystoning and other distortions that occur when written text is viewed from an angle.

The camera module is used in combination with a smart phone or other digital handheld device to provide a convenient and easy to use reading aid. Because the camera module is associated with a smart phone or other handheld digital device, the low-vision aid is in possession of the user almost all the time which facilitates convenience.

According to another example embodiment the built-in stand may include a base in the form of a rectangular ring that permits the objective of the camera to view materials within the ring when the stand is placed on top of for example printed matter.

The orientation of the illumination source located adjacent, for example below, the camera module objective facilitates high contrast in uniform illumination of the material being viewed. In addition, the illumination is positioned to mitigate reflected glare by illuminating the printed materials to be read from an angle.

According to example embodiments of the invention, the camera module may be coupled to a custom fitted protective case for various phone models or other digital device modules.

In addition, a universal stand may be suitable and adaptable to almost any handheld digital device.

### BRIEF DESCRIPTION OF THE DRAWINGS

Subject matter hereof may be more completely understood in consideration of the following detailed description of various embodiments in connection with the accompanying figures, in which:
FIG. 1 is a perspective view of a low vision aid camera module, according to an example embodiment;
FIG. 2 is a block diagram of a system for providing low vision aid capabilities, according to an embodiment;
FIG. 3 is a flowchart of a method for establishing an isolated connection between a mobile device and a low vision aid camera module, according to an embodiment;
FIG. 4 is another perspective view of a low vision aid camera module, according to an embodiment;
FIG. 5 is another perspective view of a low vision aid camera module, according to an embodiment;
FIG. 6 is a perspective view of the low-vision aid camera module depicting a foldable stand, an illumination source, and an objective lens in a swiveled position to mitigate distortion or keystoning;
FIG. 7 is a perspective view of the low-vision aid camera module according to an example embodiment with a lit illumination source and with the objective lens in a swiveled position illustrating an example field of illumination;
FIG.8 is a perspective view of a user utilizing the low-vision aid camera module illustrating an example text enhancement;
FIG. 9 is a plan view of a smart phone case that can be coupled to the low-vision aid camera module and to a smart phone according to an example embodiment of the invention; and
FIG. 10 is a perspective view of a low-vision camera module coupled to a smart: case which is in turn coupled to a smart phone further illustrating the stand in an unfolded orientation and the swiveled objective lens that is operably coupled to the stand.

While various embodiments are amenable to various modifications and alternative forms, specifics thereof have been shown by way of example in the drawings and will be described in detail. It should be understood, however, that the intention is not to limit the claimed inventions to the particular embodiments described. On the contrary, the intention is to cover all modifications, equivalents, and alternatives falling within the spirit and scope of the subject matter as defined by the claims.

### DETAILED DESCRIPTION

Smartphone applications and cameras are primarily optimized for capturing images and videos at a distance, which poses a challenge for individuals who need to focus on near-field objects, especially those with low vision. The limitations of standard smartphone cameras and apps in this regard can make it difficult for users to capture and view close-up details with clarity. To address this gap, systems and methods for a low vision aid camera module are described and contemplated herein. The low vision aid camera module can supplement the accessibility capabilities of a mobile device, such as a smartphone, to, for example, enlarge printed text.

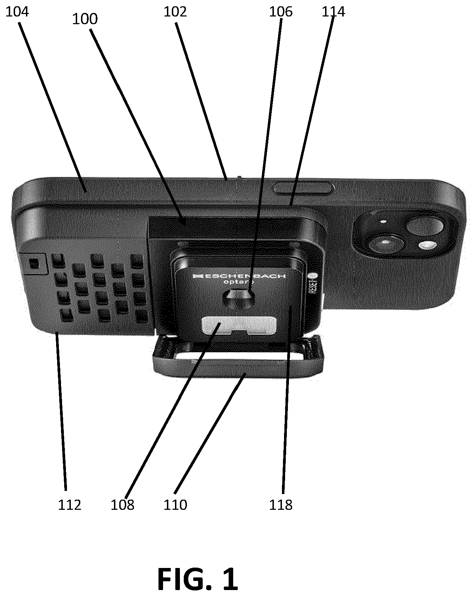

Referring to FIGs. 1, 4, 6 and 10, perspective views of a low vision aid camera module 100 are depicted according to example embodiments.

Referring particularly to FIG. 1, low-vision camera module 100 is depicted coupled to smart phone 102 via smart phone case 104. This application will refer to the use of camera module 100 in association with a smart phone 102. This should not be considered limiting as camera module 100 may be utilized with other portable digital devices such as tablets. As illustrated, low vision aid camera module 100 generally includes camera 106, illumination source 108, folding stand 110 and processor housing 112. Processor housing 112 encloses processor (not visible) and other internal components.

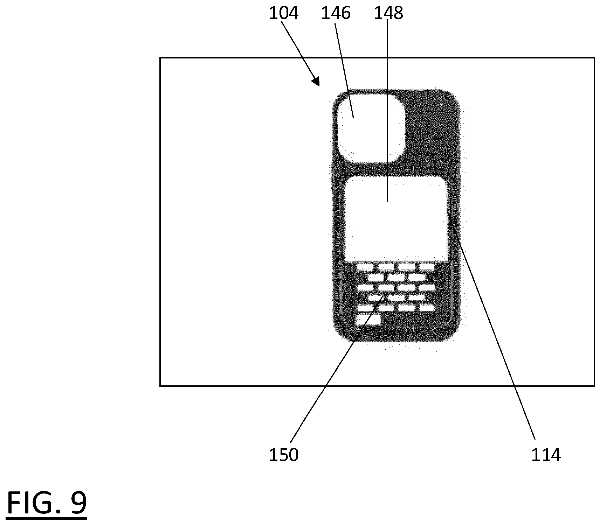

Smart phone case 104 depicted in FIG. 9, is generally similar to known smart phone cases but includes coupling structures 114 adapted to conform to and facilitate coupling between camera module 100 and smart phone 102.

Camera 106 is particularly structured and adapted to operate at close range and to have a short MOD. Camera 106 includes objective lens 116 which is structured to swivel by being operably coupled to folding stand 110. When folding stand 110 is in a folded orientation, objective lens 116 is swiveled to a position so that its optical axis is substantially perpendicular to front face 118 of camera module 100. This facilitates handheld operation of camera module in combination with smartphone 102. When folding stand 110 is in an unfolded orientation, objective lens 116 is swiveled downwardly to a non-perpendicular orientation relative to the front face to mitigate distortion and keystoning of images captured of, for example, printed material.

Keystoning is a phenomenon that occurs when images are skewed because the line of sight of the camera is not perpendicular to the surface being imaged. When keystoning occurs, a rectangle appears to be a trapezoid shape similar to the keystone in an archway, thus the name. Tilting the objective lens so that its optical axis is perpendicular or close to perpendicular to the surface being imaged to mitigates keystoning of the captured image. According to another example embodiment, both objective lens 116 and image sensor (not depicted) swivel together so that objective lens 116 remains perpendicular to image sensor (not depicted) while imaging.

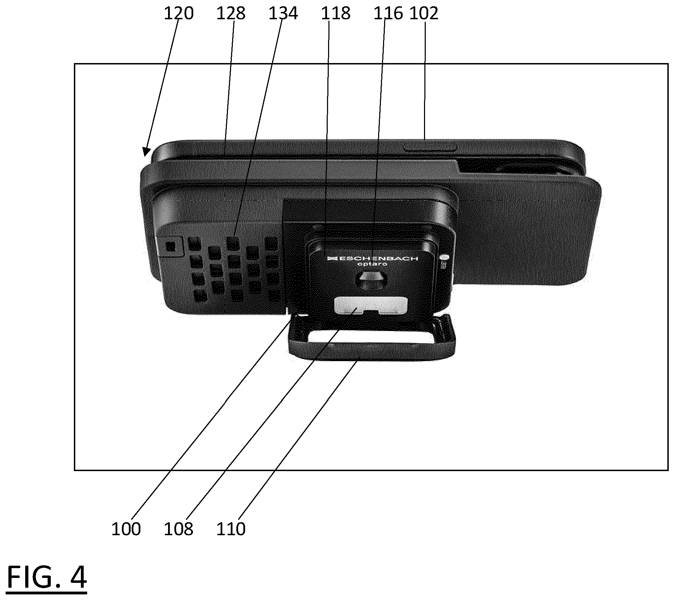

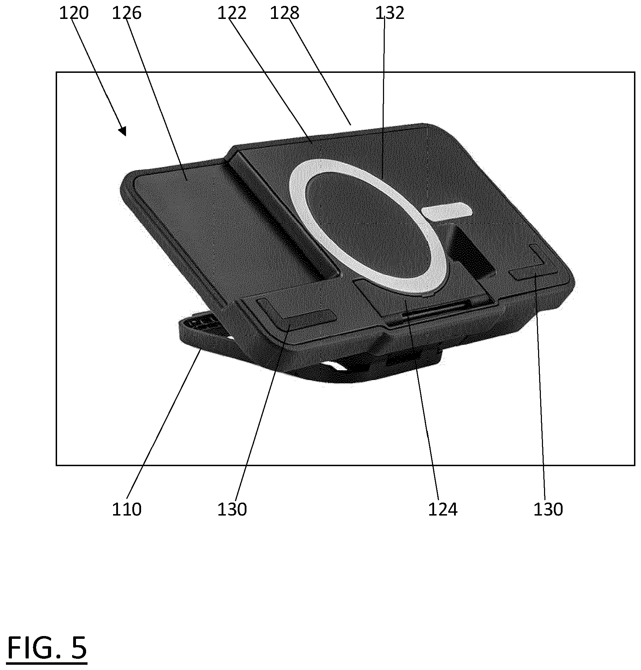

Referring particularly to FIGs. 4 and 5, according to a further example embodiment camera module 100 can further be coupled to universal stand 120.

Universal stand 120 is adapted to accommodate nearly any smart phone 102 or other digital device. Universal stand 120 further includes device platform 122, foldable shelf 124 and recessed platform 126.

It is notable that device platform 122 allows a device placed thereon to extend beyond edges 128 of device platform 122 thus accommodating digital devices of various sizes and shapes without restriction. Foldable shelf 124 can be folded flat as depicted in FIG. 5 or unfolded to extend outwardly, for example perpendicularly, from device platform 122 thereby partially supporting a device placed on device platform 122. Foldable shelf 124 also facilitates keeping a device at a substantially right angle relative to device platform 122.

Device platform 122, according to an example embodiment may include friction pads 130, for example L-shaped friction pads 130 to assist in holding a device steady and to mitigate slipping between the device and device platform 122.

Device platform 122 may further include magnetic portions 132 to further facilitate holding a device thereon.

Referring particularly to FIG. 4, camera module 100 may further present cooling apertures 136.

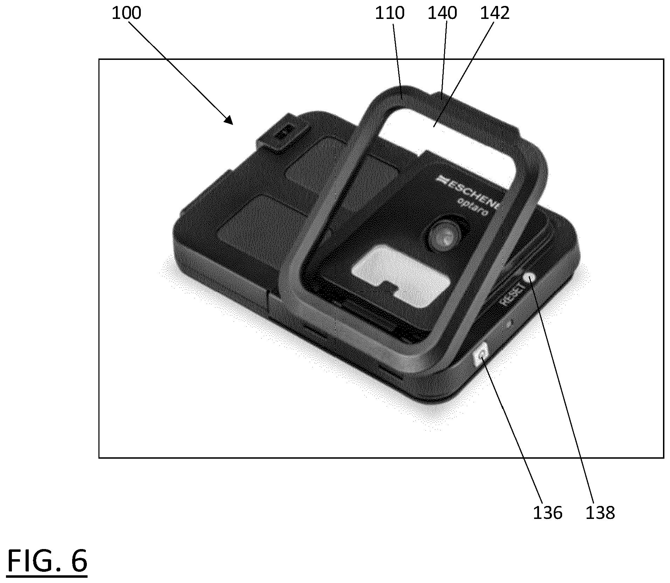

Referring particularly to FIG. 6, camera module 100, according to an example embodiment, further presents on-off switch 136 and reset button 138 on an exterior surface thereof. With further reference to FIG. 6 folding stand 110, according to an example embodiment, includes rectangular perimeter ring 140. It is apparent from FIG. 6 that objective lens 116 is positioned so that an optical axis thereof passes through rectangular opening 142 whether folding stand 110 is in an unfolded or folded orientation.

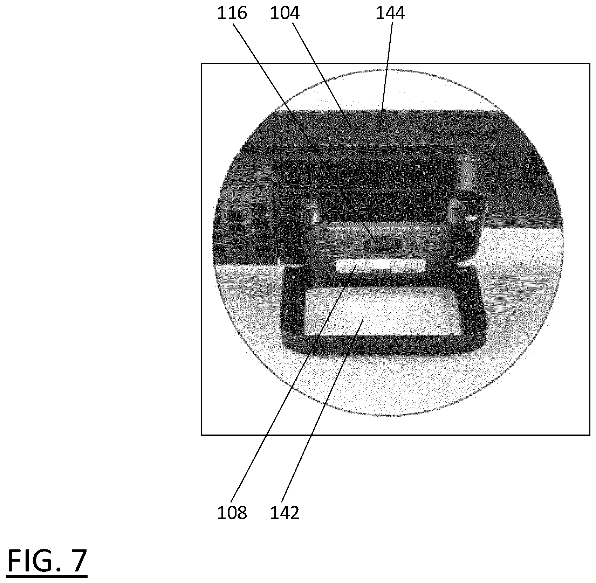

Referring particularly to FIG. 7, illumination source 108 is, for example, located below objective lens 116 and illuminates an area within rectangular opening 142 from an oblique angle that is different from the angle at which an optical axis of objective lens 116 is directed through rectangular opening 142. This enables illumination of objects or printed material in such a way as to be viewed via objective lens 116 substantially without reflected glare because specular reflected glare is directed away from objective lens 116.

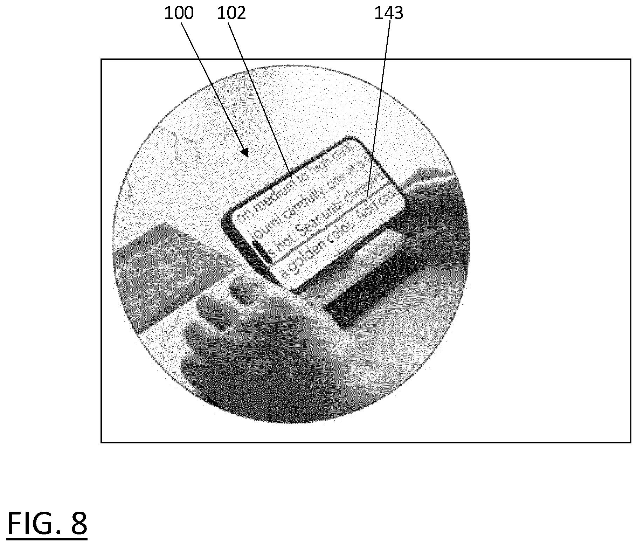

Referring particularly to FIG. 8, an example of visual enhancement 143 of an image is depicted. While many visual enhancements 143 may be utilized, in this example a red line underscores a particular line of text thus facilitating reading by visually impaired persons. Many other visual enhancements 143 may be utilized as is known to those skilled in the arts of electronic magnification. Visual enhancements include but are not limited to increased contrast between text and background, changes in the color of text, background or both, underscoring selected text or reverse contrast and combinations thereof.

Referring particularly to FIG 9, an example embodiment of smart phone case 104 is depicted. Smart phone case 104 is in many ways conventional but is particularly structured and adapted to be readily couplable to camera module 100. In the depicted embodiment smart phone case 104 includes body 144 which defines smart phone camera aperture 146, camera module coupling aperture 148 and ventilation openings 150. Smart phone case 104 can be configured in many different sizes and shapes to accommodate many different smart phones, tablets and other digital devices.

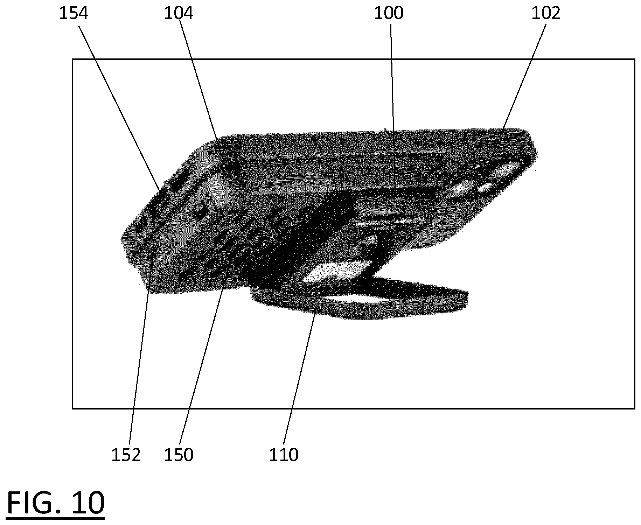

Referring to FIG. 10, camera module 100 coupled to smart phone case 104 is depicted. In FIG. 10, charging port 152 of camera module 100 is visible. Notably charging port 152 of camera module 100 is located separately but in close proximity to charging port 154 of smart phone 102 when placed in smart phone case 104. This arrangement facilitates easy connection of charging equipment to both smart phone 102 and camera module 100 either individually or simultaneously.

In example embodiments, the low vision aid camera module 100 includes illumination source 108. For example, low vision aid camera module 100 can incorporate illumination source as depicted or as for example an array of LED lights positioned around objective lens 116. Illumination source 108 illuminates the field of view of camera 106, improving visibility of objects by reducing shadows or glare that could obscure details. In some embodiments, control of illumination source 108 can be automatic based on operation of camera module 100 or determined based on environmental characteristics. To facilitate best imaging for low vision users, illumination source 108 is substantially brighter than typical illumination capabilities of a smartphone or other handheld device.

In example embodiments, low vision aid camera module 100 includes ergonomic elements, such as buttons or rubber grips. ergonomic elements can provide a comfortable grip and facilitate adjustment of settings. For example, ergonomic elements can include tactile buttons or switches that are easy to locate and use (e.g., due to size, color, texture, and the like), even for individuals with limited vision or dexterity.

In embodiments, the low vision aid camera module 100 is paired with a dedicated application (e.g., low vision aid platform) on smart phone 102 or another mobile device. The dedicated application can be used to control camera module 100 and/or manage the Wi-Fi connection. For example, the dedicated application can automatically switch the mobile device's Wi-Fi between a standard LAN connection and an isolated connection provided by the low vision aid camera module (e.g., based on a signal received from the low vision aid camera module). This seamless integration simplifies activation and deactivation of low vision aid camera module 100 as no user input may be required.

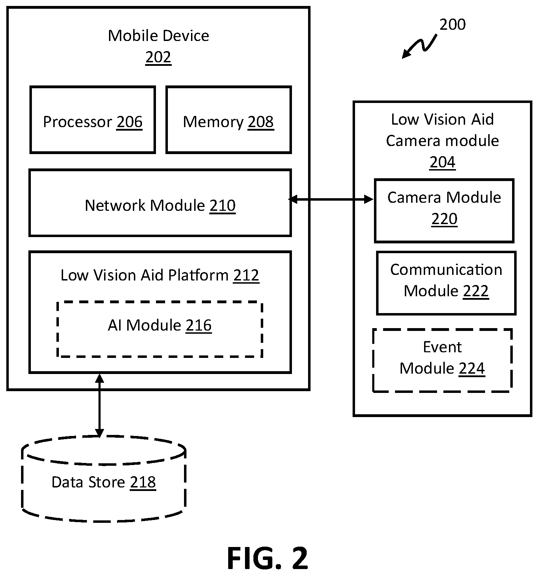

Referring to FIG. 2, a block diagram of a system 200 for providing low vision aid capabilities is depicted, according to an example embodiment. System 200 comprises mobile device 202 and low vision aid camera module 100, 204. Low vision aid camera module 204 integrates with and provides smart phone 102 or mobile device 202 with specialized near-field imaging functionality while maintaining portability and convenience of smart phone 102 or mobile device 202. System 200 can therefore be used as a comprehensive tool for users who require enhanced near-field vision capabilities.

Smart phone 102 or mobile device 202 comprises an electronic device in communication with system 200. In an example, mobile device 202 can be a smartphone, a laptop computer, a tablet, or an Internet-of-things (IoT) device, among other electronic devices. In example embodiments, mobile device 202 can be utilized by a user to interact with other components of system 200, such as retrieving a user profile from optional data store 218.

Smart phone or mobile device 202 generally includes processor 206, memory 208, network module 210, and low vision aid platform 212.

Embodiments described herein include various modules, each of which is constructed, programmed, configured, or otherwise adapted, to autonomously carry out a function or set of functions. The term module as used herein is defined as a real-world device, component, or arrangement of components implemented using hardware, such as by an application specific integrated circuit (ASIC) or field-programmable gate array (FPGA), for example, or as a combination of hardware and software, such as by a microprocessor system and a set of program instructions that adapt the engine to implement the particular functionality, which (while being executed) transform the microprocessor system into a special-purpose device. A module can also be implemented as a combination of the two, with certain functions facilitated by hardware alone, and other functions facilitated by a combination of hardware and software. In certain implementations, at least a portion, and in some cases, all, of a module can be executed on the processor(s) of one or more computing platforms that are made up of hardware (e.g., one or more processors, data storage devices such as memory or drive storage, input/output facilities such as network interface devices, video devices, keyboard, mouse or touchscreen devices, etc.) that execute an operating system, system programs, and application programs, while also implementing the module using multitasking, multithreading, distributed (e.g., cluster, peer-peer, cloud, etc.) processing where appropriate, or other such techniques.

An (e.g., each) module can be realized in a variety of physically realizable configurations and should generally not be limited to any particular implementation exemplified herein, unless such limitations are expressly identified. In addition, a module can itself be composed of sub-modules, each of which can be regarded as a module in its own right. Moreover, in the embodiments described herein, each of the various modules corresponds to a defined functionality; however, it should be understood that in other contemplated embodiments, each functionality can be distributed to more than one module. Likewise, in other contemplated embodiments, multiple defined functionalities can be implemented by a single module that performs those multiple functions, possibly alongside other functions, or distributed differently among a set of modules than specifically illustrated in the examples herein.

Network module 210 supports networking and input/output capabilities of smart phone 102 or mobile device 202. Smart phone 102 or mobile device 202 can communicate with another communication apparatus mounted with a WiFi module via the network module 210. Mobile device 202 may directly communicate with another communication apparatus, or may communicate therewith through a wireless access point, via network module 210. For example, network module 210 is configured to establish a Wi-Fi connection with another device and to send and/or receive network traffic to the other device over the Wi-Fi connection.

Low vision aid platform 212 provides mobile device 202 with a communication protocol to interact with (e.g., command) low vision aid camera module 204. In an example embodiment, low vision aid platform 212 can provide an interface, such as a graphical user interface, configured to display related event topic fields and schemas and receive user input. For example, a user can record video data received from low vision aid camera module 204 through low vision aid platform 212.

Low vision aid platform 212 can include graphical or text-based interfaces for defining and customizing near-field imaging. In an embodiment, low vision aid platform 212 generates a user profile to track the preferred parameters and use of low vision aid camera module 100, 204. In an embodiment, low vision aid platform 212 can associate the user profile with mobile device 202 such that the user profile is automatically implemented when mobile device 202 connects with any low vision aid camera module 100. In an embodiment, low vision aid platform 212 can associate the user profile with low vision aid camera module 100, 204 such that the user profile is implemented on any mobile device connecting with low vision aid camera module 100, 204.

In an embodiment, low vision aid platform 212 integrates with other systems, applications, and databases to access data, trigger events, and exchange information. Connectors, APIs, and adapters can allow for interoperability and further processing of video data received from low vision aid camera module 100, 204. For example, low vision aid platform 212 can access an optional data store 218. Data store 218 can be used to store user profiles, operational parameters, or software updates.

In an embodiment, as illustrated in FIG. 2, low vision aid platform 212 is implemented on a smart phone 102 or single mobile device 202, having its own processor 206 and memory 208. In embodiments, low vision aid platform 212 can be a cloud-based service such that customization and execution of low vision aid camera module workflows can be stored or executed on a remote server or across a network of multiple computing devices (e.g., with each device having its own processor and memory).

Optional AI module 216 is configured to utilize AI models to refine near-field capabilities of low vision aid camera module 100, 204. For example, AI module 216 can monitor the implementation of image modifiers, such as contrast characteristics, and log the details to personalize handling of data from low vision aid camera module 100, 204 (e.g., for mobile device 202).

In an embodiment, training and use data associated with operation of low vision aid camera module 100, 204 can be stored in data store 218 such that statistics can be gathered for later monitoring and updating purposes.

In an example embodiment, properties associated with near-field camera use cases can be updated for future implementation. In particular, AI module 216 can implement a feedback loop where the application of modifiers (e.g., magnification level, contrast settings, brightness) are used to automatically apply appropriate modifiers across similar use cases. For instance, if a setting is frequently adjusted in certain conditions (e.g., when viewing black text on a white page) but not in others, AI module 216 can learn to associate the applicability of the setting with the determined environmental factors (e.g., prevalence of white and black pixels above a threshold).

With continued reference to FIG. 2, data store 218 can comprise one or more storage repositories, such as a database, logical disk space, file, or other suitable storage medium configured to store workflow elements. In an embodiment of a database, data store 106 can be a general-purpose database management storage system (DBMS) or relational DBMS as implemented by, for example, ORACLE, IBM DB2, Microsoft SQL Server, PostgreSQL, MySQL, SQLite, LINUX, or UNIX solutions.

Low vision aid camera module 100, 204 may be coupled to or incorporated with a protective smart phone case 104 for mobile device 202 that provides improved near field viewing capabilities by incorporating advanced optical and technological features. Low vision aid camera module 100, 204 can be configured to removably couple mobile device 202. For example, low vision aid camera module 100, 204 can be sized and structured based on the dimensions and external access ports of mobile device 202. Low vision aid camera module 100, 204 generally includes a camera module 220, a communications module 222, and an optional event module 224.

Camera module 220 can include a macro camera configured to optimize image capture at close range (e.g., from 1/2 to 6 inches, 1.25-15 cm). Camera 106 provides higher magnification, resolution and clarity than the standard camera on smart phone 102 or mobile device 202, allowing users to see magnified fine details that might otherwise be difficult to discern.

Communication module 222 is configured to emit a Wi-Fi signal, allowing low vision aid camera module 100, 204 to directly send video data to and receive instructions from network module 210 of mobile device 202. The direct connection allows transmission of video data captured by camera module 220 with maximal fidelity and minimal latency. Additionally, the Wi-Fi connection can be established between the low vision aid camera module 100. 204 and smart phone102 or mobile device 202 without reliance on external network availability.

Event module 224 is configured to process events and notify network module 210 on event detection. For example, event module 224 can act as an event manager that monitors the state and operational parameters of camera module 220 or other elements of low vision aid camera module 100, 204 during use. In such an embodiment, event module 224 can track the status, environmental data, and/or metadata associated with each use instance, allowing for real-time monitoring, reporting, and adjustment.

In an example embodiment, event module 224 can be used in conjunction with other elements of low vision aid camera module 100, 204, such as communication module 222. For example, in an embodiment of low vision aid camera module 100, 204 that includes a built-in folding stand 110 connection status (e.g., over a private Wi-Fi connection) can be based on a detected orientation state of folding stand 110. If event module 224 determines folding stand 110 is in an opened or unfolded orientation, communication module 222 can disable mobile device 202's connection to any existing Wi-Fi networks (LAN) and establish an exclusive connection using the Wi-Fi channel provided by communication module 222. This isolated connection allows camera 106 to securely transmit high-quality, close-up images to smart phone 102 or mobile device 202 and mitigates the risk of the download capacity of mobile device 202 being throttled by data from other sources.

When folding stand 110 is closed, the Wi-Fi channel maintained by communication module 222 can be deactivated. In embodiments, smart phone 102 or mobile device 202 can automatically reconnect to previously used Wi-Fi networks. The exclusive Wi-Fi channel can also be manually activated by user selection based on an app to facilitate handheld use.

In operation, system 200 can be used for activities that require close-up visual inspection, such as reading small print, examining photographs, or inspecting small objects. System 200 provides an all-in-one solution for users with low vision, combining the portability and functionality of a mobile device with the specialized capabilities of an advanced visual aid.

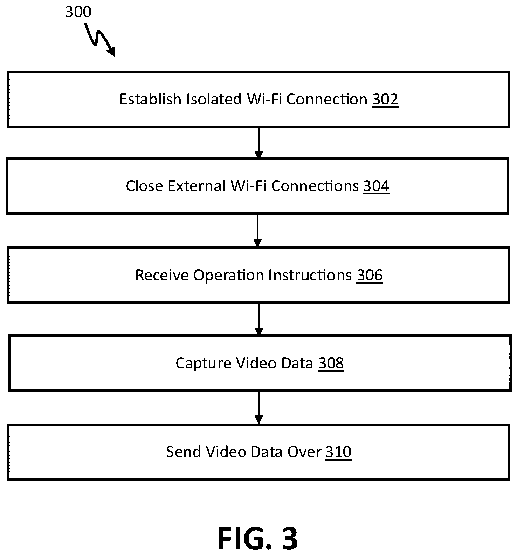

Referring to FIG. 3, a flowchart of a method 300 for establishing an isolated connection between a smart phone 102 or mobile device 202 and low vision aid camera module 100, 204 is depicted, according to an embodiment. In an embodiment, method 300 can be implemented by system 200 as depicted in FIG. 2.

At 302, method 300 comprises establishing an isolated Wi-Fi connection between smart phone 102 or mobile device 202 and the low vision aid camera module 100. A communication module of the low vision aid camera module creates a dedicated Wi-Fi network solely for communication with smart phone 102 or mobile device 202.

At 304, method 300 comprises closing external Wi-Fi connections of the mobile device. When the isolated connection is established, the communication module effectively isolates smart phone 102 or mobile device 202 from other Wi-Fi networks while the isolated connection is active. This isolation allows for a stable, secure, and interference-free link between the camera module 100 and smart phone 102 or mobile device 202.

At 306, method 300 comprises the low vision aid camera module 100, 204 receiving operation instructions from smart phone 102 or mobile device 202 over the isolated Wi-Fi connection. For example, smart phone 102 or mobile device 202 might send commands to adjust camera 106 settings, activate illumination source 108, or start and stop video capture.

At 308, method 300 comprises capturing video data with a camera module of the low vision aid camera module 100, 204. In embodiments, video data can be processed (e.g., modifiers can be applied) by low vision aid camera module 100, 204.

At 310, method 300 comprises sending processed video data from low vision aid camera module 100, 204 to smart phone 102 or mobile device 202. The direct transmission of video data facilitates timely and uninterrupted delivery of the video feed.

Embodiments of the present disclosure include low vision camera module 100, 204 configured to optimize image capture at close range (e.g., macro camera 106). The camera 106 module is seamlessly incorporated into the body of the low vision aid camera module 100, 204. In some implementations, low vision aid camera module 100, 204 is powered by an integrated independent battery, preserving battery life of smart phone 102 or mobile device 202 while maintaining the performance of the close-range camera 106. The independent battery can then be charged via charging port 152 when the user would normally charge smart phone 102 or mobile device 202 via charging port 154, allowing for extended periods of use without draining smart phone 102 or mobile device 202 resources or requiring the user to keep track of multiple devices.

Embodiments of the present disclosure include communication module 222. Communication module 222 is configured to emit a Wi-Fi signal, allowing low vision aid camera module 100, 204 to communicate directly with smart phone 102 or mobile device 202 (e.g., without relying on the device's previously established Wi-Fi or local area network (LAN) connections). The direct connection allows transmission of video data captured by the camera 106 with minimal latency and maximal fidelity. Additionally, connection can be established between the low vision aid camera module 100, 204 and smart phone 102 or mobile device 202 without reliance on external network availability.

Embodiments of the present disclosure include event module 224 configured to detect the occurrence of (pre)defined events associated with the low vision aid camera module 100, 204. For example, in some implementations low vision aid camera module 100, 204 includes a built-in folding stand 110. The event module can determine whether folding stand 110 is in an unfolded orientation and take actions accordingly.

If folding stand 110 is in an unfolded orientation, event module 224 can automatically activate low vision camera module 100, 204. Simultaneously, event module 224 can prompt communication module 222 to disable any existing network connections smart phone 102 or mobile device 202 may have and establish an exclusive connection.

When folded stand 110 is in a folded orientation, event module 224 can deactivate camera module 220 and/or communication module 222, and smart phone 102 or mobile device 202 can automatically reconnect to its previous Wi-Fi network. This seamless transition between connections allows users to efficiently switch between some near-field camera functions and mobile device functionality without needing to manually adjust their phone's settings.

The figures and foregoing description relate to embodiments by way of illustration only. It should be noted that from the foregoing discussion, alternative embodiments of the structures, systems, and methods disclosed herein will be readily recognized as viable alternatives that may be employed without departing from the principles of what is claimed.

## Claims

1. A low vision aid system including a low vision aid camera module to be used with a smart phone or a mobile device, comprising:
a macro camera configured to focus on near-field objects, the macro camera being integrated within the low vision aid camera module;
an illumination source structured to illuminate a field of view associated with the macro camera;
a battery power supply independent of the smart phone or mobile device; and
a wireless communication module configured to:
establish an isolated Wi-Fi connection to the mobile device, wherein the isolated Wi-Fi connection prevents the mobile device from communicating over other Wi-Fi networks while the isolated Wi-Fi connection is maintained,
receive, via the isolated Wi-Fi connection, instructions from the smart phone or the mobile device, wherein the instructions are associated with operation of the macro camera, and
send, via the isolated Wi-Fi connection, a video bitstream to the mobile device based on the instructions.

2. The low vision aid system of claim 1, wherein the low vision aid camera module further includes an integrated stand, wherein the integrated stand is shiftable between a folded orientation and an unfolded orientation.

3. The low vision aid system of claim 2, wherein the integrated stand is operably coupled to an objective lens of the macro camera so that the objective lens swivels to an angled orientation when the integrated stand is shifted to the unfolded orientation thereby mitigating at least one of distortion and keystoning of printed material viewed.

4. The low vision aid system of claim 1, wherein the low vision aid camera module is removably coupled to or integrated into a smart phone case or a mobile digital device case structured to at least partially surround the smart phone or the mobile digital device.

5. The low vision aid system of claim 2, wherein the integrated stand includes a perimeter structure at least partially surrounding a central opening through which an optical axis of the macro camera passes in at least one of the folded and unfolded orientation.

6. The low vision aid system of claim 1, further comprising a universal stand that is couplable to the low vision aid camera module and that enables a selected smart phone or digital device to be attached to the camera module via the universal stand for use as an integrated unit.

7. The low vision aid system of claim 6, further wherein the universal stand includes magnets that couple to the smart phone or the digital device magnetically.

8. The low vision aid system of claim 1, wherein the macro camera has a minimum object distance (MOD) or working distance to facilitate placement of the device close to the text or other object to be viewed in a range of 1.25 to 15 centimeters.

9. The low vision aid system of claim 1, wherein an objective lens of the macro camera is incorporated into the camera module so that the objective lens is centrally located centrally relative to a screen display of the smart phone or the digital device when the smart phone or the digital device is coupled to the smart phone or the digital device.

10. The low vision aid system of claim 1, wherein the camera module is configured to include image signal processing whereby visual enhancements of a captured image are performed to assist users with impaired vision or wherein the camera module displays the images at a magnification of 3.3 x to 15 x.

11. A method of establishing an isolated Wi-Fi connection between a mobile device and a low vision aid camera module, comprising:
closing external Wi-Fi connections of the mobile device;
creating a dedicated Wi-Fi network solely for communication with the mobile device thereby effectively isolating the mobile device from other Wi-Fi networks while the isolated connection is active;
receiving operation instructions at the low vision aid camera module from the mobile device over the isolated Wi-Fi connection;
capturing video data by operation of the low vision aid camera module; and
sending the video data from the low vision aid camera module to the mobile device.

12. The method as claimed in claim 11, further comprising processing the video data prior to the sending.

13. A method of utilizing a low vision aid, comprising:
coupling a low vision aid camera module to a digital electronic device having a display screen by application of a dedicated, isolated Wi-Fi connection that excludes other Wi-Fi connections while the dedicated, isolated Wi-Fi connection is operating;
receiving at the low vision aid camera module via the dedicated, isolated Wi-Fi connection, instructions from the digital electronic device to the low vision aid camera module, wherein the instructions are associated with operation of the low vision aid camera module;
capturing video images of objects near a camera of the low vision aid camera module with the low vision aid camera module;
transmitting the video images from the low vision aid camera module to the digital electronic device in a video bitstream; and
displaying the images on the display screen.

14. The method as claimed in claims 13, further comprising processing the video images at the low vision aid camera module prior to transmitting the video images to the digital electronic device to be displayed on the display screen.

15. The method as claimed in claim 13, further comprising shifting an integrated stand of the low vision aid camera module between a folded orientation and an unfolded orientation.

16. The method as claimed in claim 13, further comprising swiveling an objective lens of the low vision aid camera module so that the objective lens swivels from a perpendicular orientation to an angled orientation when the integrated stand is shifted to the unfolded orientation thereby mitigating at least one of distortion and keystoning of printed material viewed.

17. The method as claimed in claim 13, further comprising coupling the digital electronic device to a universal stand that enables a selected smart phone or digital device to be attached to the camera module for use as an integrated unit.

18. The method as claimed in claim 13, further comprising applying image signal processing to perform visual enhancements of a captured image to assist users with impaired vision or displaying the images at a magnification of 3.3 x to 15 x.

19. The method as claimed in claim 13, further comprising utilizing a minimum object distance (MOD) or working distance to facilitate placement of the to the text or other object to be viewed in a range of 1.25 to 15 centimeters.

20. The method as claimed in claim 13, further comprising removably coupling the low vision aid camera module to a mobile digital device case structured to at least partially surround the mobile device.
